(19) Europäisches Patentamt / European Patent Office / Office européen des brevets

(11) **EP 2 432 798 B1**

(12) **EUROPÄISCHE PATENTSCHRIFT**

(45) Veröffentlichungstag und Bekanntmachung des Hinweises auf die Patenterteilung:
**03.04.2013 Patentblatt 2013/14**

(21) Anmeldenummer: **10735198.3**

(22) Anmeldetag: **07.07.2010**

(51) Int Cl.:
***C07J 31/00*** *(2006.01)* ***C07J 41/00*** *(2006.01)*
***A61K 31/567*** *(2006.01)* ***A61P 5/36*** *(2006.01)*

(86) Internationale Anmeldenummer:
**PCT/EP2010/004149**

(87) Internationale Veröffentlichungsnummer:
**WO 2011/009531 (27.01.2011 Gazette 2011/04)**

(54) **17-HYDROXY-17-PENTAFLUORETHYL-ESTRA-4,9(10)-DIEN-11-ARYL-DERIVAT, VERFAHREN ZU SEINE HERSTELLUNG UND SEINE VERWENDUNG ZUR BEHANDLUNG VON KRANKHEITEN**

17-HYDROXY-17-PENTAFLUORETHYL-ESTRA-4,9(10)-DIEN-11-ARYL DERIVATIVE, METHODS FOR THE PRODUCTION THEREOF AND USE THEREOF FOR TREATING DISEASES

DÉRIVÉ 17-HYDROXY-17-PENTAFLUORÉTHYL-ESTRA-4,9(10)-DIÈNE-11-ARYLÉS, SON PROCÉDÉ DE PRODUCTION ET SA UTILISATION POUR LE TRAITEMENT DE MALADIES

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**BA ME RS**

(30) Priorität: **20.07.2009 DE 102009034362**

(43) Veröffentlichungstag der Anmeldung:
**28.03.2012 Patentblatt 2012/13**

(60) Teilanmeldung:
**12194455.7**

(73) Patentinhaber: **Bayer Intellectual Property GmbH**
**40789 Monheim (DE)**

(72) Erfinder:
- **SCHWEDE, Wolfgang**
  **16548 Glienicke (DE)**
- **KLAR, Ulrich**
  **13503 Berlin (DE)**
- **MÖLLER, Carsten**
  **10115 Berlin (DE)**
- **ROTGERI, Andrea**
  **13503 Berlin (DE)**
- **BONE, Wilhelm**
  **13467 Berlin (DE)**

(74) Vertreter: **BIP Patents**
**c/o Bayer Intellectual Property GmbH**
**Creative Campus Monheim**
**Alfred-Nobel-Straße 10**
**40789 Monheim (DE)**

(56) Entgegenhaltungen:
**WO-A1-98/34947** **US-A- 4 609 651**
**US-A- 4 900 725** **US-A- 5 712 264**
**US-A1- 2002 143 000**

Bemerkungen:
Die Akte enthält technische Angaben, die nach dem Eingang der Anmeldung eingereicht wurden und die nicht in dieser Patentschrift enthalten sind.

EP 2 432 798 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft das 17-Hydroxy-17-pentafluorethyl-estra-4,9(10)-dien-11-aryl Derivat der Formel A mit Progesteron antagonisierender Wirkung und Verfahren zu seiner Herstellung, seine Verwendung zur Behandlung und/oder Prophylaxe von Krankheiten sowie seine Verwendung zur Herstellung von Arzneimitteln zur Behandlung und/oder Prophylaxe von Krankheiten, insbesondere von Uterusfibroiden (Myomen, uterine Leiomyome), der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption.

Diese Verbindung ist ein wertvoller pharmazeutischer Wirkstoff. Sie kann unter anderem zur Herstellung pharmazeutischer Präparate zur Behandlung von Uterusfibroiden oder der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption verwendet werden. Zur Behandlung von Uterusfibroiden und der Endometriose kann die erfindungsgemäße Verbindung auch sequentiell in Kombination mit Gestagenen verabreicht werden. In einem solchen Behandlungsregime könnte die efindungsgemäße Verbindung über einen Zeitraum von 1 - 6 Monaten gegeben werden, gefolgt von einer Behandlungspause oder einer sequentiellen Behandlung mit einem Gestagen über einen Zeitraum von 2 - 6 Wochen oder gefolgt von der Behandlung mit einem oralen Kontrazeptivum (OC-Kombinationen) über den gleichen Zeitraum.

Die Wirksamkeit der erfindungsgemäßen Verbindung als Progesteronrezeptorantagonist wurde in vitro in Transaktivierungstests und in vivo an der Ratte (Terminierung der frühen Schwangerschaft) gezeigt. Verbindungen mit antagonistischer Wirkung am Progesteronrezeptor (kompetitive Progesteronrezeptorantagonisten) sind erstmals 1982 bekannt (RU 486; EP57115) und seither zahlreich beschrieben worden. Progesteronrezeptorantagonisten mit fluorierter 17α-Sitenkette wurden in WO 98/34947 und Fuhrmann et al., J. Med. Chem. 43, 5010 - 5016 (2000) veröftentlicht.

Die in WO 98/34947 beschriebenen Verbindungen mit fluorierter 17α-Seitenkette weisen im allgemeinen eine sehr starke antagonistische Aktivität am Progesteronrezeptor auf. Sehr potente und daher in WO 98/34947 bevorzugte Verbindungen sind 11β-(4-Acetylphenyl)-20,20,21,21,21-pentafluor-17-hydroxy-19-nor-17α-pregna-4,9-dien-3-on, 11β-(4-Acetylphenyl)-20,20,21,21,21-pentafluor-17-hydroxy-19-nor-17α-pregna-4-en-3-on und 6'-Acetyl-9,11β-dihydro-17β-hydroxy-17α-(1,1,2,2,2-pentafluorethyl)-4'H-naphth[3',2',1':10,9,11]ester-4-en-3-on. Diese Verbindungen werden in vivo in erheblichem Maße in verschiedene Metabolite überführt, die zum Teil starke, zum Teil geringere pharmakologische Aktivität aufweisen. Der Metabolismus tritt überwiegend am 4-Substituenten des 11-Phenylrestes auf. In WO 2008/058767 werden Verbindungen beschrieben, die zumindest zum Teil Metabolite der in WO 98/34947 beschriebenen Verbindungen sind.

Aufgabe der vorliegenden Erfindung ist es einen hoch potenten kompetitiven Progesteronrezeptorantagonisten zur Verfügung zu stellen und damit alternative Behandlungsmöglichkeiten gynäkologischer Erkrankungen zu schaffen.

Es wurde gefunden, dass die erfindungsgemäße Verbindung A besonders geeignet ist um diese Aufgabe zu lösen. Sie zeigt eine sehr starke antagonistische Aktivität am Progesteronrezeptor, d.h. sie inhibiert die Wirkung des Progesterons an seinem Rezeptor.

Ferner wurde gefunden, dass die Verbindung A im Vergleich z.B. zu den Alkanoylgruppen eine wesentlich höhere metabolische aber auch chemische Stabilität gegenüber Temperatur, Licht und oxidativem Stress aufweisen. Die Verbindung (11β,17β)-17-hydroxy-11-[4-(methylsulfonyl)phenyl]-17-(pentafluorethyl)estra-4,9-dien-3-on (Beispiel 4) zeigt verglichen mit dem jeweiligen Analogon mit einer Alkanoyl- oder Hydroxyalkanoylgruppe (11β-(4-Acetylphenyl)-20,20,21,21,21-pentafluor-17-hydroxy-19-nor-17a-pregna-4,9-dien-3-on bzw. 20,20,21,21,21-Pentafluor-17-hydroxy-11β-[4-(hydroxyacatyl)phenyl]-19-nor-17β-pregna-4,9-dien-3-on) eine überraschend hohe Stabilität bei thermischer Belastung, unter Einfluss von UV-Licht und eine überraschend geringe Oxidationsempfindlichkeit.

Überraschend ist auch, dass die Verbindungen A, ein geringes Inhibrtionspotential aufweisen bezüglich der untersuchten CYP-Isoenzyme CYP1A2, CYP2C8, CYP2C9, CYP2D6 und CYP3A4 und bis zu einer maximal im Assay löslichen bzw eingesetzten Konzentration (mindestens 10 μM, maximal 20 μM) in keinem untersuchten Fall eine 50%ige Inhibition erreicht wurde.

Diese in vitro Befunde lassen für die untersuchte Substanz ein besonders niedriges Risiko für Interaktionen mit co-administrierten Arzneimitteln hinsichtlich CYP-Inhibition vermuten.

Ferner konnte für (11β,17β)-17-hydroxy-11-[4-(methylsulfonyl)phenyl]-17-(pentafluorethyl)estra-0,9-dien-3-on in präklinischen Untersuchungen an Nagem und Nicht-Nagem ein besonders günstiges Sicherheitsprofil (in akuten und chonischen Versuchen) gefunden werden.

Die vorliegende Erfindung betrifft das obengenannte 17-Hydroxy-17-pentafluorethyl-estra-4,9(10)-dien-11-aryl-Derivat (d.h. Verbindung A) mit der chemischen Formel A.

(A)

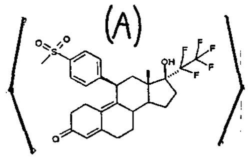

"Verbindung A" und die erfindungsgemäße Verbindung" schließen die obengenannte Verbindung und seine Salze, Solvate oder Solvate der Salze, einschließlich aller Kristallmodifikafionen ein.

Als <u>Salze</u> sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindung umfassen - wenn eine basische Funktion enthalten ist - Salze mit anorganischen oder organischen Säuren, insbesondere von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindung umfassen - wenn eine saure Funktion enthaltend ist - Alkalimetallsalze, Erdalkalimetallsalze oder Ammoniumsalze, wie sie durch Umsetzung mit entsprechenden anorganischen oder organischen Basen erhalten werden können. Beispielhaft und vorzugsweise seien genannt Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin, N-Methylpiperidin, N-Methyl-glukamin, D-Methyl-glukamin, Ethyl-glukamin, 1,6-Hexadiamin, Glukosamin, N-Methylglycin, 2-Amino-1,3-propandiol, Tris-hydroxy-methyl-aminomethan und 1-Amino-2,3,4-butantriol. Als <u>Solvate</u> werden im Rahmen der Erfindung solche Formen der erfindungsgemäßen Verbindung bezeichnet, welche in festem oder flüssigem Zustand eine Adduktbildung mit Lösungsmittelmolekülen zeigen. Das Lösungsmittel kann dabei in einem stöchiometrischen oder auch unstöchiometrischen Verhältnis vorliegen. Bei stöchiometrischen Solvaten spricht man auch von Hemi-, (Semi-), Mono-, Sesqui-, Di-, Tri-, Tetra-, Penta-, usw. Solvaten. Hydrate sind eine spezielle Form der Solvate, bei denen die Koordination mit Wasser erfolgt.

Im Rahmen der vorliegenden Beschreibung haben die Substituenten, soweit nicht anders spezifiziert, die folgende Bedeutung:

<u>Alkyl</u> steht für gerad- oder verzweigtkettige Alkylgruppen mit 1-6 Kohlenstoffatomen, wie beispielsweise Methyl, Ethyl, Propyl, Isopropyl, n-Butyl, sec.-Butyl, Isobutyl, tert.-Butyl, n-Pentyl, Isopentyl, Neopentyl Hexyl, Heptyl, Octyl, Nonyl und Decyl.

<u>Aryl</u> steht für einen mono- bis tricyclischen aromatischen, substituierten oder unsubstituierten carbocyclischen Rest, wie zum Beispiel Phenyl, Naphthyl, die einfach oder mehrfach mit Halogen (F, Cl, Br, I), OH, O-Alkyl, $CO_2H$, $CO_2$-Alkyl, $NH_2$, $NH(C_1$-$C_{10}$-Alkyl), $N(C_1$-$C_{10}$-Alkyl$)_2$, insbesondere $N(CH_3)_2$, $NO_2$, $N_3$, CN, $C_1$-$C_{10}$-Alkyl, $C_1$-$C_{10}$-Perfluor-Alkyl, $C_1$-$C_{10}$-Acyl, $C_1$-$C_{10}$-Acyloxy-Gruppen, substituiert sein können. <u>Heteroaryl</u> steht für einen aromatischen, mono- oder bicyclischen Rest mit in der Regel 5 bis 10, vorzugsweise 5 bis 6 Ringatomen und bis zu 5, vorzugsweise bis zu 4 Heteroatomen aus der Reihe S, O und N, beispielhaft und vorzugsweise für Benzofuranyl, Benzothiophenyl, Chinolinyl, Furyl, Imidazolyl, Indazolyl, Indolyl, Isochinolinyl Oxazolyl, Pyridazinyl, Pyridyl, Pyrimidyl, Pyrrolyl, Thiazolyl, Thienyl, Pyrazolyl, Isoxazolyl, Pyrazinyl, Chinolyl oder Tetrazolyl.

<u>Aralkyl</u> steht für Aralkylgruppen, die im Ring bis zu 14 Kohlenstoffatome, bevorzugt 6-10 Kohlenstoffatome, und in der Alkylkette 1-8, bevorzugt 1-4, Kohlenstoffatome enthalten können. Als Aralkylreste kommen beispielsweise Benzyl, Phenylethyl, Naphthylmethyl, Naphthylethyl, Furylmethyl, Thienylethyl, Pyridylpropyl in Betracht. Die Ringe können einfach oder mehrfach durch Halogen, OH, O-Alkyl, $CO_2H$, $CO_2$Alkyl, $NH_2$, $NH(C_1$-$C_{10}$-Alkyl), $N(C_1$-$C_{10}$-Alkyl$)_2$, $NO_2$, $N_3$, CN, $C_1$-$C_{20}$-Alkyl, $C_1$-$C_{10}$-Perfluor-Alkyl, $C_1$-$C_{20}$Acyl, $C_1$-$C_{20}$-Acyloxy-Gruppen substituiert sein.

Es wurde gefunden, dass die efindungsgemäße Verbindung eine gute, das Progesteron antagonisierende Wirkung aufweisen. In mehreren klinischen Studien wurde gefunden, dass die Behandlung mit Progestronrezeptorantagonisten (Mifepriston, Asoprisnil, Proellex) zu einer signifikanten Schrumpfung von Uterusfibroiden und einer signifikanten Reduktion der mit diesen Uterusfibroiden assoziierten Symptome führen kann. Ferner wurde in klinischen Studien gezeigt, dass unter einer Behandlung mit den genannten Progesteronrezeptorantagonisten auch die von Endometriose

verursachten Symptome (insbesondere Schmerzen) deutlich reduziert werden können.

Schema 1

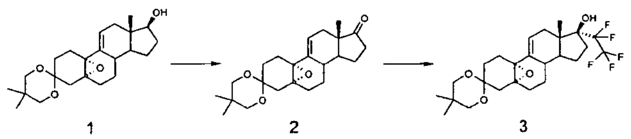

1 2 3

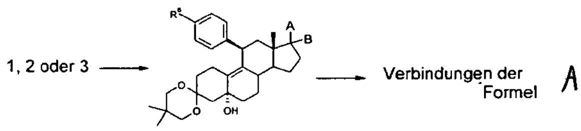


Verbindungen der allgemeinen Formel II

(A und B haben in der oben genannten Formel die folgende Bedeutung: =O; -OH/-H oder -OH/-$C_2F_5$)

Eine Übersicht zur Herstellung von Verbindungen der Formel A ist in schema 1 gezeigt. Die Darstellung der Verbindung mit der chemischen Formel A erfolgt ausgehend von (5'R,8'S,10'R,13'S,14'S,17'S)-5,5,13'-Trimethyl-1',2',7',B',12',13',14',15',16',17-decahydro-6'H-spiro[1,3-dioxan-2,3'-[5,10]epoxycyclopenta[a]phenanthren)-17'-ol (Herstellung siehe Tetrahedron Lett. 26, 2069-2072 (1985) in Analogie zu den in WO 98/34947 und in WO 2008/058767 beschriebenen Verfahren. Nach Oxidation der Hydroxygruppe in Position 17 des Steroidgerüstes erfolgt die Einführung der 17α-Pentafluorethylseitenkette an die entsprechenden 17-Ketoverbindungen gemäß den in WO 98/34947 und in WO 2008/058767 beschriebenen Verfahren. Die Einführung der 11β-Phenylsubstituenten erfolgt über konjugierte Addition von Arylgrignard- oder Aryllithiumreagenzien unter Kupfer-Katalyse. Hierbei werden Verbindungen der allgemeinen Formel II erhalten, in denen $R^8$ alle bereits für $R^1$ genannten Bedeutungen haben kann und zusätzlich eine Hydroxy-, $C_1$-$C_{10}$-Alkoxy-, Benzyloxy-, $C_1$-$_{10}$Alkanoyloxy-, Benzoyloxy-, Silyloxyl-, Alkoxyalkyloxygruppe ein Cl, Br, I oder eine Gruppierung $C_mF_m$+1,$SO_3$ mit m=1-4 sein kann und A bzw. B entweder für eine Carbonylgruppe stehen oder für eine 17β-OH/17α-H Gruppierung oder für eine 17β-OH/17α-C2F5 Gruppierung steht. Aus Verbindungen der allgemeinen Formel II kann dann die Verbindung der Formel A erhalten werden. Hierzu werden funktionelle Gruppen gegebenenfalls weiter modifiziert. Hierbei sind insbesondere die Oxidation der Sulfide zu Sulfonen nach den dem Fachmann bekannten Methoden zu nennen. Alternativ hierzu können aber auch dem Fachmann bekannte Verfahren ausgehend von entsprechenden Sulfoxid genutzt werden. Generell kann sowohl der 11β-Phenylrest sowie auch die 17β-Pentafluorethylseitenkette zuerst eingeführt werden. Funktionelle Gruppen, besonders die 3-Ketogruppe werden gegebenenfalls zwischenzeitlich, z.B. als Ketal geschützt. Als Ketalschutzgruppen sind beispielsweise die Ethylendioxy- oder die 2,2-Dimethylpropylen-1,2-dioxygruppe zu nennen. Hydroxygruppen werden beispielsweise in Form von Methoxymethyl-, Methoxy-ethyl-, Tetrahydropyranyl-, Benzyl-, oder Silylethem geschützt.

Auf einer geeigneten Stufe erfolgt dann die Abspaltung der Schutzgruppen nach den dem Fachmann bekannten Verfahren. Bei der Spaltung des 3-Ketals zur 3-Ketogruppe des Steroidgerüstes wird eine gegebenenfalls noch vorhandene 5α-Hydroxygruppe elimininiert, so dass Verbindung A entsteht.

Soweit die Herstellung der Ausgangsverbindungen hier nicht beschrieben ist, sind diese dem Fachmann bekannt oder analog zu bekannten Verbindungen oder hier beschriebenen Verfahren herstellbar. Die Isomerengemische können nach üblichen Methoden, wie beispielsweise Kristallisation, Chromatographie oder Salzbildung in die einzelnen Verbindungen aufgetrennt werden.

Die Herstellung der Salze erfolgt in üblicher Weise, indem man eine Lösung der Verbindung A mit der äquivalenten Menge oder einem Überschuss einer Base oder Säure, die sich gegebenenfalls in Lösung befindet, versetzt, gegebenenfalls den Niederschlag abtrennt oder in üblicher Weise die Lösung aufarbeitet.

Die resultierende Verbindung der Formel A wird gebenenfalls mit den entsprechenden (i) Lösungsmitteln und/oder (ii) Basen oder Säuren zu ihren Solvaten, Salzen und/oder Solvaten der Salze umsetzt.

Die oben aufgeführten allgemeinen oder in Vorzugsbereichen angegebenen Restedefinitionen gelten sowohl für das Endprodukte der Formel A als auch entsprechend für die jeweils zur Herstellung benötigten Ausgangsstoffe bzw. Zwischenprodukte.

Die erfindungsgemäße Verbindung zeigt ein nicht vorhersehbares, wertvolles pharmakologisches, pharmakokinetisches und pharmakodynamisches Wirkprofil.

Sie eignet sich daher zur Verwendung als Arzneimittel zur Behandlung und/oder Prophylaxe von Krankheiten bei Menschen und Tieren.

Die pharmazeutische Wirksamkeit der erfindungsgemäßen Verbindung lässt sich durch seine Wirkung als Progesteronrezeptorantagonist, also seine antagonierende Wirkung am Progesteronrezeptor erklären. Weiterer Gegenstand der vorliegenden Erfindung ist der Einsatz der erfindungsgemäßen Verbindung zur Behandlung und/oder Prophylaxe von Erkrankungen basierend auf hormonabhängigen hyperproliferativen Prozessen, vorzugsweise von gynäkologischen Krankheiten, insbesondere von Uterusfibroiden, der Endometriose oder von hormonabhängigen Mammakarzinomen.

Weiterer Gegenstand der vorliegenden Erfindung ist die Verwendung der erfindungsgemäßen Verbindung zur Behandlung und/oder Prophylaxe von Erkrankungen, insbesondere der zuvor genannten Erkrankungen.

Weiterer Gegenstand der vorliegenden Erfindung ist die erfindungsgemäße Verbindung zur Verwendung in einem Verfahren zur Behandlung und/oder Prophylaxe von Uterusfibroiden, der Endometriose und von hormonabhängigen Mammakarzinomen.

Weiterer Gegenstand der vorliegenden Erfindung ist die Verwendung der erfindungsgemäßen Verbindung zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist Verbindung A zur Behandlung und/oder Prophylaxe von Erkrankungen, insbesondere der zuvor genannten Erkrankungen, unter Verwendung von 0,1-100 mg der erfindungsgemäßen Verbindung pro Tag und Patientin bei der Behandlung von Uterusfibroiden oder der Endometriose und für die kontrazeptive Anwendung bzw. von 0,1-500 mg der erfindungsgemäßen Verbindung pro Tag und Patientin bei Tumor-Erkrankungen (z.B. Menginiome oder hormonabhängige Tumore wie z.B. das Mammakarzinom) und bei der Notfallkontrazeption.

Weiterer Gegenstand der vorliegenden Erfindung sind Arzneimittel, enthalten die erfindungsgemäße Verbindung und mindestens einen oder mehrere weitere Wirkstoff, insbesondere zur erfindungsgemäße Verbindung und mindestens einen oder mehrere weitere Wirkstoffe, insbesondere zur Behandlung und/oder Prophylaxe der zuvor genannten Erkrankungen.

Zur Behandlung von Tumor-Erkrankungen können z.B. die folgenden Wirkstoffe/Wirkstoffklassen entweder gleichzeitig oder sequentiell verabreicht werden: SERMs, SERDs, Antiöstrogene, Aromataseinhibitoren, Kinaseinhibitoren, Angiogeneseinhibitoren und/oder Cytostatika.

Zur Behandlung von Uterusfibroiden oder der Endometriose können die erfindungemäße Verbindung gleichzeitig oder sequentiell mit Gestagenen oder Kombinationen aus Östrogenen und Gestagenen kombiniert werden.

In WO 96/15794 (Spicer et al., Balance Pharm. Inc.), WO 96/03130 (Stöckemann et al., Schering AG) und PCT/EP2009/003249 (Möller et al., Bayer Schering Pharma AG) sind Progesteronerezeptorantagonisten/Gestagen-Regime offenbart. Für die Behandlung von Uterusfibroiden und der Endometriose gut geeignet sind - sich gegebenenfalls wiederholende - Regime in denen der Progesteronrezeptorantagonist über einen Zeitraum von zwei bis vier Monaten gegeben wird, gefolgt von der Gabe des Gestagens über einen Zeitraum von ein bis vier Wochen. Besonders gut geeignet ist die - sich gegebenenfalls wiederholende - 84tägige Gabe des Progesteronrezeptorantagonisten gefolgt von der 14tägigen Gabe des Gestagens.

Zur Behandlung von mit der Menopause assoziierten Beschwerden kommt eine gleichzeitige oder sequentielle Verabreichung der erfindungsgemäßen Verbindung z.B. mit SERMs, SERDs und Östrogenen in Betracht.

SERMs (Selective Estrogen Receptor Modulators) sind solche Verbindungen, die gewebeseletiv entweder eine antiestrogene bzw. estrogene Wirkung haben, beispielsweise am Uterus die Wirkung des Östrogens inhibieren, am Knochen aber eine neutrale oder dem Östrogen ähnliche Wirkung haben. Beispiele sind Clomifen, Raloxifen, Tamoxifen, Torimifen, Bazedoxifen, Lasofoxifen und Ormeloxifen.

Selektive Estrogenrezeptordestabilisatoren (SERD) sind Arzneistoffe, die den Estrogenrezeptor vollständig antagonisieren (‚reine Antiöstrogene' ohne östrogene Wirkkomponente) und zu einem Abbau des Rezeptors führen (beispielsweise Fulvestrant, ZK-703 und ZK-253 (Hoffmann J et al., J Natl Cancer Inst 2004, 96:210-218) sowie in WO 98/007740, WO 99/33855 und WO 03/045972 beschriebene Verbindungen.

Antiöstrogene sind Verbindungen die den Estrogenrezeptor vollständig antagonisieren, beispielsweise Fulvestrant.

Aromataseinhibitoren inhibieren das Enzym Aromatase und somit die Aromatisierung von Androgenen in Estrogene. Dazu gehören u.a. Anastrozole, Letrozole, Exemestane, Vorozole, Formestane and Fadrozole. Kinaseinhibitoren sind Enzyme, die einen Phosphatrest von ATP auf andere Substrate, dort insbesondere auf Hydroxygruppen, übertragen, z.B. Sorafenib (Nexavar) oder Imatinib (Gleevec). Angiogenesehemmer, z.B. Avastin, reduzieren bzw. blockieren die Gefäßversorgung und damit die Durchblutung eines Tumors.

Zytostatika, z.B. cis-Platin, Taxol, Taxotere sind natürliche oder synthetische Substanzen, die das Zellwachstum bzw. die Zellteilung hemmen.

Als Gestagene werden im Sinne vorliegender Erfindung entweder das natürliche Progesteron selbst verstanden oder synthetische Derivate, die wie das Progesteron selbst an den Progesteronrezeptor binden und in Dosierungen, die über der Ovulationshemmdosis liegen, die Ovulation hemmen. Als Beispiele für die synthetischen Derivate seien das Drospirenon, Gestoden, Levonorgestrel, Cyproteronacetat, Desogestrel und 3-Ketodesogestrel, Norethisteron, Norethisteronacetat und das Dienogest genannt.

Bei Kombinationen aus Gestagenen und Östrogenen handelt es sich um die Wirkstoffkombinationen, die in den an sich bekannten oralen Kontrazeptiva, beispielsweise Yasmin, Femovan, Triquilar, Marvelon, YAZ etc., enthalten sind.

Die erfindungsgemäße Verbindung kann systemisch und/oder lokal wirken. Zu diesem Zweck kann sie auf geeignete Weise, wie z.B. oral, intrauterin[är], intravaginal, parenteral, pulmonal, nasal, sublingual, lingual, buccal, rectal, dermal, transdermal, conjunctival, otisch oder als Implantat bzw. Stent appliziert werden.

Intrauterin[är] bedeutet dabei insbesondere die Applikation mittels IUS (intrauterine system) oder IUD (intrauterine device). Die intravaginale Applikation kann u.a. mittels IVRNRS (Intra-Vaginalring/Vaginalringsystem) erfolgen.

Intrauterine oder intravaginale Applikationsformen (vergl. z.B. WO 01/47490. insbesondere Seite 1, Zeile 10 bis Seite 5, Zeile 13 und Seite 7, Zeile 19 bis Seite 58, Zeile 6, oder für Vaginalringe: WO 06/010097, insbesondere Seite 10, Zeile 22 bis Seite 14, Zeile 28) können dabei die erfindungsgemäße Verbindung und Nonsilikon- und/oder Silikonpolymere, insbesondere auch siloxanebasierte Elastomere (vergl. WO 01/47490, insbesondere Seite 7, Zeile 19-Seite 15, Zeile 15), enthalten.

Für diese Applikationswege können die erfindungsgemäße Verbindung in geeigneten Applikationsformen verabreicht werden.

Für die orale Applikation eignen sich nach dem Stand der Technik funktionierende schnell und/oder modifiziert die erfindungsgemäße Verbindung abgebende Applikationsformen, die die erfingdungsgemäße Verbindung in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäße Verbindung kann in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien. Weiterer Gegenstand der vorliegenden Erfindung sind Arzneimittel, die die erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofem nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

Die folgenden Beispiele 1-3 und 5-15 sind Vergleichsbeispiele. Beispiel 4 ist ein Erfindungsbeispiel.

Die folgenden Beispiele dienen der Erläuterung der Erfindung ohne diese in irgend einer Weise zu beschränken.

**Beispiel 1:**

(11β,17β)-17-hydroxy-11-[4-(methylsulfanyl)phenyl]-17-(pentafluorethyl)estra-4,9-dien-3-on

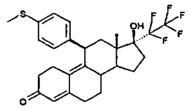

a) (5'R,8'S,10'R,13'S,14'S,17S)-5,5,13'-Trimethyl-17'-(pentafluorethyl)-1',2',7',8',12',13',14',15',16',17'-decahydro-6'H-spiro[1,3-dioxan-2,3'-[5,10]epoxycyclopenta[a]phenanthren]-17'-ol

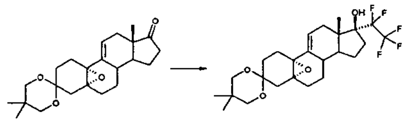

Zu 116 g kondensiertem Pentafluoriodethan in 500 ml absolutem Toluol wurden bei -70°C 50 g (5'R,8'S,10'R, 13'5,14'S)-5,5,13'-Trimethyl-1',2',6',7',8',12',13',14',15',16'-decahydro-17'H-spiro[1,3-dioxan-2,3'-[5,10]epoxycyclopenta[a]phenanthren]-17'-on (Herstellung siehe Tetrahedron Lett. 26, 2069-2072 (1985) gegeben. Dazu gab man 290 ml einer 1,5 molaren Lösung von Methyllithium-Lithiumbromid-Komplex in Diethylether bei gleicher Temperatur. Anschließend wurde eine Stunde bei 0°C nachgerührt. Dann wurde das Reaktionsgemisch auf gesättigte wässrige Ammoniumchloridlösung gegeben und mit Ethylacetat extrahiert. Die organische Phase wurde mit gesättigter wässriger Natriumchloridlösung gewaschen, über Natriumsulfat getrocknet und im Vakuum eingeengt. Das Rohprodukt wurde in 200 ml Aceton aufgelöst und mit 450 ml Wasser versetzt. Das ausgefallene Produkt wurde abfiltriert und im Vakuum getrocknet.
Ausbeute 61,6 g
1H-NMR (400 MHz, CDCl3): δ= 6,04 brd (1H); 3,60 d (1H): 3,35-3,50 m (3H); 2,51 dbr (1H); 1,06 s (3H); 0,93 s (3H); 0,85 s (3H).

b) (5R,8S,11R,13S,14S,17S)-5',5',13-Trimethyl-11-[4-(methylsulfanyl)phenyl]-17-(pentafluorethyl)-1,2,6,7,8,11,12,13,14,15,16,17-dodecahydrospiro[cyclopenta[a]phenanthren-3,2'-[1,3]dioxan]-5,17(4H)-diol

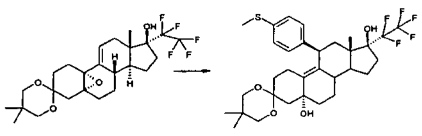

1,23 g Magnesium-Späne wurden in 5 ml THF suspendiert und unter Rühren mit 50 μl Dibromethan versetzt. Zur Suspension wurde eine Lösung von 10,31 g 1-Brom-4-(methylthiopheny)benzol in 60 ml THF so addiert, dass die Reaktionstemperatur nicht über 55 °C stieg. Danach wurde eine Stunde nachgerührt. Dann wurde die entstandene Lösung auf 0 °C gekühlt. Man addierte 151 mg CuCl und ließ weitere 15 Minuten bei 0°C nachrühren. Danach wurde eine Lösung von 5 g der unter Beispiel 1a) beschriebenen Substanz in 50 ml THF addiert. Dann ließ man das Reaktionsgemisch unter Rühren über ca. 3 Stunden auf 23°C kommen und rührte anschließend bei dieser Temperatur 10 Stunden nach. Anschließend wurde gesättigte wässrige $NH_4Cl$-Lösung zum Reaktionsgemisch unter externer Kühlung addiert. Man rührte weitere 30 Minuten nach und extrahierte dann mehrfach mit Ethylacetat. Die vereinigten organischen Phasen wurden mit gesättigter Natriumchloridlösung gewaschen und über Natriumsulfat getrocknet. Das Rohprodukt wurde durch Chromatographie an Kieselgel und anschließende Kristallisation aus einem Gemisch von Dichlormethan und Diisopropylether aufgereinigt. Man erhielt 5,72 g der Titelverbindung.
$^1$H-NMR (300 MHz, $CDCl_3$): δ= 7,50 d (2H); 7,30 d (2H); 4,41 s (1H); 4,28 dbr (1H); 3,40-3,60 m (4H); 2,51 s (3H); 1,05

s (3H); 0,87 s (3H); 0,53 s (3H).

c) (11β,17β)-17-hydroxy-11-[4-(methylsulfanyl)phenyl]-17-(pentafluorethyl)estra-4,9-dien-3-on

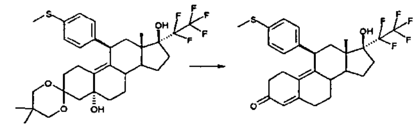

500 mg der unter 1b) beschriebenen Verbindung wurden in 15 ml Methanol gelöst. Man addierte 360 μl halbkonzentrierte Schwefelsäure und ließ 3 Stunden bei 23°C nachrühren. Danach wurde das Reaktionsgemisch auf gesättigte wässrige Natriumhydrogencarbonatlösung gegossen. Man extrahierte mehrfach mit Ethylacetat. Die vereinigten organischen Phasen wurden mit gesättigter wässriger Natriumchloridlösung gewaschen, über Natriumsulfat getrocknet und im Vakuum eingeengt. Das Rohprodukt wurde durch Chromatographie an Kieselgel gereinigt. Man erhielt 297 mg der Titelverbindung. $^1$H-NMR (300 MHz, $CDCl_3$): δ= 7,20 d (2H); 7,13 d (2H); 5,80 sbr (1H); 4,45 dbr (1H); 2,51 s (3H); 0,68 s (3H).

**Beispiel 2**

(11β,17β)-11-(4-(Ethylsulfanyl)phenyl)-17-hydroxy-17-(pentafluorethyl)estra-4,9-dien-3-on

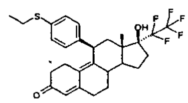

a) (5R,8S,11R,13S,14S,17S)-11-[4-(Ethylsulfanyl)phenyl]-5',5',13-trimethyl-17-(pentafluorethyl)-1,2,6,7,8,11,12,13,14,15,16,17-dodecahydrospiro[cyclopenta[a]phenanthren-3,2'-[1,3]dioxan]-5,17(4H)-diol

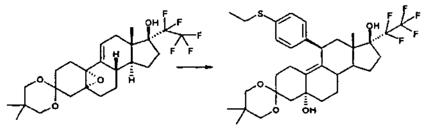

Analog zu Beispiel 1b) wurden aus 3 g der unter 1a) beschriebenen Verbindung, 888 mg Magnesium-Spänen, 91 mg CuCl und 7,94 g 1-Brom-4-(Ethylthiopheny)benzol in THF 2,7 g der Titelverbindung hergestellt.
$^1$H-NMR (300 MHz, $CDCl_3$): δ= 7,50 d (2H); 7,38 d (2H): 4,43 s (1H); 4,39 dbr (1H); 3,40-3,60 m (3H); 2,95 q (2H); 1,30 t (3H); 1,07 s (3H); 0,87 s (3H); 0,53 s (3H).

b) (11β,17β)-11-[4-(Ethylsulfanyl)phenyl]-17-hydroxy-17-(pentafluorethyl)estra-4,9-dien-3-on

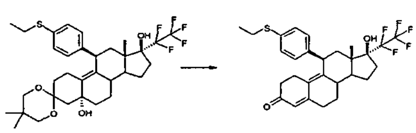

Analog zu Beispiel 1c) wurden aus 200 mg der unter 2a) hergestellten Verbindung durch Umsetzung mit

halbkonzentrierte Schwefelsäure in Methanol 125 mg der Titelverbindung hergestellt.
$^1$H-NMR (300 MHz, $CDCl_3$): δ= 7,21 d (2H); 7,08 d (2H); 5,78 sbr (1H); 4,43 dbr (1H); 2,93 q (2H); 1,29 t (3H); 0,60 s (3H).

**Beispiel 3**

(11β,17β)-17-Hydroxy-11-{4-[(RS)-methylsulfinyl]phenyl}-17-(pentafluorethyl)estra-4,9-dien-3-on

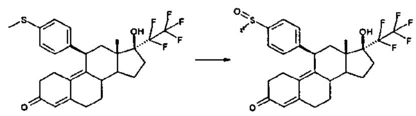

Zu 0,5 ml Trifluoressigsäure wurden bei 23°C 180 μl 30%ige Wasserstoffperoxidlösung addiert. Man rührte 30 Minuten nach und addierte dann das Gemisch zu einer auf 10°C gekühlten Suspension von 533 mg der unter Beispiel 1c) hergestellten Verbindung in 1,8 ml Trifluoressigsäure. Es wurde 2 Stunden bei 10°C nachgerührt. Danach wurde das Reaktionsgemisch auf Eiswasser gegossen. Man ließ 2 Stunden nachrühren und filtrierte danach das ausgefallene Produkt ab. Das erhaltene Rohprodukt wurde durch Chromatographie an Kieselgel gereinigt. Man erhielt 146 mg der Titelverbindung sowie 123 mg der unter Beispiel 4 beschriebenen Verbindung.
$^1$H-NMR (400 MHz, $CDCl_3$): δ= 7,58 d (2H); 7,38 d (2H); 5,80 sbr (1H); 4,50 dbr (1H); 2,71 s (3H); 0,58 s (3H) + 0,56 s (3H) (Mischung der Diastereomere).

**Beispiel 4**

(11β,17β)-17-Hydroxy-11-[4-(methylsulfonyl)phenyl]-17-(pentafluorethyl)estra-4,9-dien-3-on

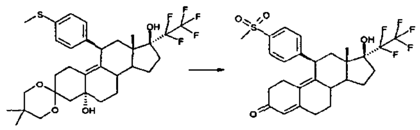

5 g der unter Beispiel 1b) beschriebenen Verbindung wurden in einem Gemisch aus 140 ml THF und 140 ml Methanol gelöst. Man tropfte bei 0°C eine Lösung von 20 g Oxone® in 94 ml Wasser langsam hinzu. Anschließend ließ man 3,5 Stunden bei 0°C nachrühren. Dann wurde das Reaktionsgemisch mit einer Mischung aus Wasser und Dichlormethan versetzt. Man trennte die Phasen und extrahierte die wässrige Phase mehrmals mit Dichlormethan. Die vereinigten organischen Phasen wurden mit gesättigter wässriger Natriumchloridlösung gewaschen, über Natriumsulfat getrocknet und im Vakuum eingeengt. Das Rohprodukt wurde durch Chromatographie an Kieselgel gereinigt. Man erhielt 3,8 g der Titelverbindung.
$^1$H-NMR (300 MHz, $CDCl_3$): δ= 7,86 d (2H); 7,40 d (2H); 5,81 sbr (1H); 4,50 dbr (1H); 3,07 s (3H); 0,51 s (3H).

**Beispiel 5**

(11β,17β)-11-[4-(Ethylsulfonyl)phenyl]-17-hydroxy-17-(pentafluorethyl)estra-4,9-dien-3-on

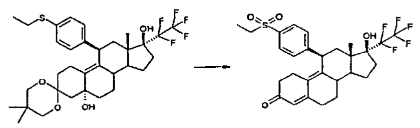

Analog zu Beispiel 4) wurden durch Umsetzung von 400 mg der unter Beispiel 2a) beschriebenen Verbindung mit 1,56 g Oxone® in einem Gemisch aus 10 ml THF und 10 ml Methanol nach Aufreinigung durch Chromatographie

an Kieselgel 183 mg der Titelverbindung erhalten.
$^1$H-NMR (400 MHz, $CDCl_3$): δ= 7,82 d (2H); 7,40 d (2H); 5,80 sbr (1H); 4,52 dbr (1H); 3,13 q (2H); 1,28 t (3H); 0,51 s (3H).

**Beispiel 6**

(11β,17β)-11-[4-(benzylsulfanyl)phenyl]-17-hydroxy-17-(pentafluorethyl)estra-4,9-dien-3-on

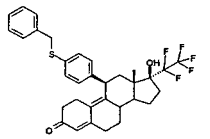

a) (5R,8S,11R,13S,14S,17S)-11-[4-(Benzylsuffanyl)phenyl]-5',5',13-Mmethyl-17-(pentafluorethyl)-1,2,6,7,8,11,12,13,14,15,16,17-dodecahydrospiro[cyclopenta[ajphenanthren-3,2'-[1,3]dioxan]-5,17(4H)-diol

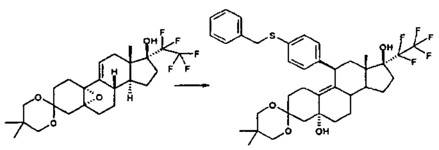

Analog zu Beispiel 1b) wurden aus 8,5 g der unter 1a) beschriebenen Verbindung, 2,64 g Magnesium-Spänen, 171 mg CuCl und 30,36 g 1-Benzylsulfanyl-4-brombenzol in THF 6,65 g der Titelverbindung hergestellt.
$^1$H-NMR (300 MHz, $CDCl_3$): δ= 7,13-7,30 m (7H); 7,10 d (2H); 4,44 s (1H); 4,27 dbr (1H); 4,05 s (2H); 3,40-3,60 m (4H); 1,05 s (3H); 0,87 s (3H); 0,51 s (3H).

b) (11β,17β)-11-[4-(benzylsulfanyl)phenyl]-17-hydroxy-17-(pentafluorethyl)estra-4,9-dien-3-on

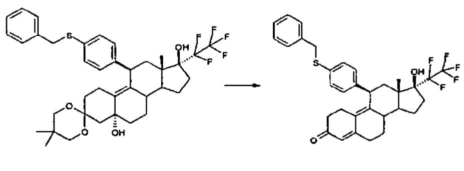

Analog zu Beispiel 1c) wurden aus 1,62 g der unter Beispiel 6a) hergestellten Verbindung durch Umsetzung mit halbkonzentrierte Schwefelsäure in Methanol 1,02 g der Titelverbindung hergestellt. $^1$H-NMR (300 MHz, $CDCl_3$): δ= 7,15-7,40 m (7H); 7,06 d (2H); 5,78 sbr (1H); 4,40 dbr (1H); 4,08 s (2H); 0,59 s (3H).

**Beispiel 7**

N-[(4-[(11β,17β)-17-hydroxy-3-oxo-17-(pentafluorethyl)estra-4,9-dien-11-yl]phenyl}(RS)(methyl)oxido-$\lambda^6$-sulfanyliden]-4-methylbenzolsulfonamid

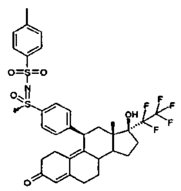

a) N-[{4-[(5R,8S,11R,13S,14S,17S)-5,17-Dihydroxy-5',5',13-trimethyl-17-(pentafluorethyl)-1,2,4,5,6,7,8,11,12,13,14,15,16,17-tetradecahydrospiro[cyclopenta[a]phenanthren-3,2'-[1,3]dioxan]-11-yl]phenyl}(RS)(methyl)-λ$^4$-sulfanyliden]-4-methylbenzolsulfonamid

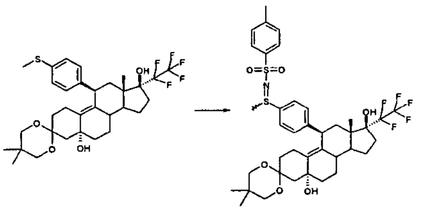

3 g der unter Beispiel 1b) beschriebenen Substanz wurden in 80 ml Acetonitril suspendiert. Man addierte 1,64 g Chloramin-T-Trihydrat® und ließ 20 Stunden bei 23°C nachrühren. Anschließend wurde das Reaktionsgemisch mit 70 ml Dichlormethan verdünnt. Man filtrierte ausgefallenes Natriumchlorid ab, und engte dann im Vakuum ein. Das Rohprodukt wurde durch Chromatographie an Kieselgel gereinigt. Man erhielt 3,16 g der Titelverbindung.
$^1$H-NMR (300 MHz, $CDCl_3$): δ= 7,74 d (2H); 7,49 d (2H); 7,38 d (2H); 7,18 d (2H); 4,40 s (1H); 4,33 dbr (1H); 3,40-3,70 m (4H); 2,80 (3H); 2,37 s (3H), 1,05 s (3H); 0,89 s (3H); 0,45 s (3H) (Mischung von Diastereomeren).

b) N-[{4-[(5R,8S,11R,13S,14S,17S)-5,17-Dihydroxy-5',5',13-trimethyl-17-(pentafluorethyl)-1,2,4,5,6,7,8,11,12,13,14,15,16,17-tetradecahydrospiro[cyclopenta[a]phenanthren-3,2'-[1,3]dioxan]-11-yl]phenyl}(RS)(methyl)oxido-λ$^6$-sulfanyliden]-4-methylbenzolsulfonamid

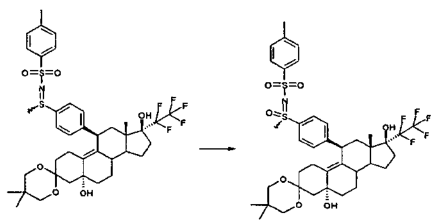

3,16 g der unter 7a) erhaltenen Verbindung wurden in 2,5 ml Acetonitril und 1,6 ml Methanol gelöst. Es wurden 1,22 g Natriumcarbonat und 2,34 ml 30%ige Wasserstoffperoxidlösung addiert. Danach ließ man 2,5 Stunden bei 23°C nachrühren. Dann wurde das Reaktionsgemisch auf Wasser gegossen. Man extrahierte mehrfach mit Dichlormethan. Die vereinigten organischen Phasen wurden mit gesättigter wässriger Natriumchloridlösung gewaschen, über Natriumsulfat getrocknet und im Vakuum eingeengt. Das Rohprodukt wurde durch Chromatographie an Kieselgel gereinigt. Man erhielt 2,56 g der Titelverbindung.
$^1$H-NMR (300 MHz, $CDCl_3$): δ= 7,78-8,00 m (4H); 7,51 d (2H); 7,31 d (2H); 4,50 s (1H); 4,44 dbr (1H); 3,45-3,67 m (7H); 2,46 s (3H); 1,09 s (3H); 0,91 s (3H); 0,51 s (3H) (Mischung von Diastereomeren).

c) N-[{4-[(11β,17β)-17-hydroxy-3-oxo-17-(pentafluorethyl)estra-4,9-dien-11-yl]phenyl}(RS)(methyl)oxido-λ$^6$-sulfanyliden]-4-methylbenzolsulfonamid

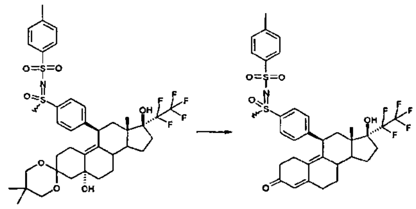

Analog zu Beispiel 1c) wurden aus 2,72 g der unter 7b) hergestellten Verbindung durch Umsetzung mit halbkonzentrierte Schwefelsäure in Methanol 2,2 g der Titelverbindung hergestellt.
$^{1}$H-NMR (300 MHz, $CDCl_3$): δ= 7,95 d (2H); 7,86 d (2H); 7,45 d (2H); 7,28 d (2H); 5,81 sbr (1H); 4,51 dbr (1H); 3,41 s (3H); 2,40 s (3H); 0,51 s (3H) (Mischung von Diastereomeren).

**Beispiel 8**

(11β,17β)-17-Hydroxy-11-[4-(RS-methylsulfonimidoyl)phenyl]-17-(pentafluoroethyl)estra-4,9-dien-3-on

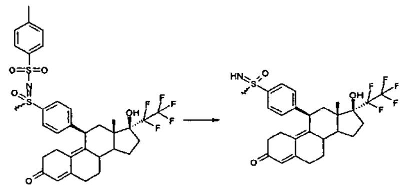

500 mg der unter Beispiel 7c) hergestellten Verbindung wurden in 10 ml Chloroform gelöst. Man addierte bei 0°C 1,15 ml konzentrierte Schwefelsäure und ließ 7 Stunden bei 0°C nachrühren. Danach wurde das Reaktionsgemisch auf gesättigte wässrige Natriumhydrogencarbonatlösung gegossen. Anschließend wurde durch Zugabe von 5%iger NaOH basisch gestellt. Man extrahierte mehrfach mit Dichlormethan. Die vereinigten organischen Phasen wurden mit gesättigter wässriger Natriumchloridlösung gewaschen, über Natriumsulfat getrocknet und im Vakuum eingeengt. Das Rohprodukt wurde durch Chromatographie an Kieselgel gereinigt. Man erhielt 306 mg der Titelverbindung.
$^{1}$H-NMR (300 MHz, $CDCl_3$): δ= 7,91 d (2H); 7,39 d (2H); 5,81 sbr (1H); 4,50 dbr (1H); 3,12 s (3H) + 3,10 s (3H); 0,56 s (3H) + 0,40 s (3H) (Mischung von Diastereomeren).

**Beispiel 9**

(11β,17β)-17-Hydroxy-11-[4'-(methylsulfanyl)biphenyl-4-yl]-17-(pentafluorethyl)estra-4,9-dien-3-on

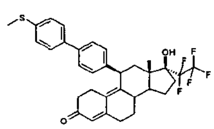

a)
(5R,8S,11R,13S,14S,17S)-11-[4-(Benzyloxy)phenyl]-5',5',13-trimethyl-17-(pentafluorethyl)-1,2,6,7,8,11,12,13,14,15,16,17-dodecahydrospiro[cyclopenta[a]phenanthren-3,2'-[1,3]dioxan]-5,17(4H)-diol

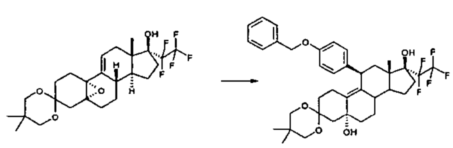

2,47 g Magnesium-Späne wurden 5 ml THF suspendiert und unter Rühren mit 50 µl Dibromethan versetzt. Zur Suspension wurde eine Lösung von 26,7 g 1-Brom-4-(phenylmethoxy)benzol in 115 ml THF bei 65 °C langsam gegeben. Die entstandene Lösung wurde auf 0 °C gekühlt. Dazu wurden 301 mg CuCl gegeben. Man rührte 10 Minuten bei 0°C nach und addierte dann langsam eine Lösung von 10 g der unter Beispiel 1a) beschriebenen Substanz in 70 ml THF. Man ließ das Reaktionsgemisch unter Rühren über ca. 3 Stunden auf 23°C kommen und rührte anschließend bei dieser Temperatur 10 Stunden nach. Anschließend wurde gesättigte wässrige $NH_4Cl$-Lösung zum Reaktionsgemisch unter externer Kühlung addiert. Man rührte weitere 30 Minuten nach und extrahierte dann mehrfach mit Ethylacetat. Die vereinigten organischen Phasen wurden mit gesättigter Natriumchloridlösung gewaschen und über Natriumsulfat getrocknet. Das Rohprodukt wurde durch Chromatographie an Kieselgel und anschließende Kristallisation aus einem Gemisch von Dichlormethan und Diisopropylether aufgereinigt. Man erhielt 9,7 g der Titelverbindung.
$^1H$-NMR (400 MHz, $CDCl_3$): δ= 7,30-7,50 m (5H); 7,12 d (2H); 6,88 d (2H); 5,02 s (2H); 4,43 s (1H); 4,28 dbr (1H); 3,50-3,60 m (3H); 3,42 d (1H); 1,06 s (3H); 0,87 s (3H); 0,56 s (3H).

b) (5R,8S,11R,13S,14S,17S)-11-[4-(Benzyloxy)phenyl]-5',5',13-trimethyl-17-(pentafluorethyl)-1,2,6,7,8,11,12,13,14,15,16,17-dodecahydrospiro[cyclopenta[a]phenanthren-3,2'-[1,3]dioxan]-5,17(4H)-diol

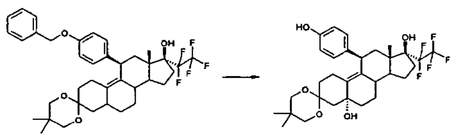

Zu einer Lösung von 9,72 g der unter 9a) beschriebenen Verbindung in 100 ml Methanol wurden 5,53 g Ammoniumformiat und 972 mg Palladium auf Aktivkohle (10%ig) addiert. Man rührte 2 Stunden bei 23°C nach und filtrierte dann über Celite®. Das Filtrat wurde im Vakuum eingeengt. Man erhielt 8,5 g Rohprodukt, welches ohne Reinigung in die Folgestufe eingesetzt wurde.
$^1H$-NMR (300 MHz, $CDCl_3$): δ= 7,05 d (2H); 6,70 d (2H); 4,43 sbr (1H); 4,27 dbr (1H); 3,50-3,58 m (3H); 3,41 sbr (1H); 1,94 s (3H); 0,86 s (3H); 0,54 s (3H).

c) 4-[(5R,6S,11R,13S,14S,17S)-5,17-Dihydroxy-5',5',13-trimethyl-17-(pentafluorethyl)-1,2,4,5,6,7,6,11,12,13,14,15,16,17-tetradecahydrospiro[cyclopenta[a]phenanthren-3,2'-[1,3]dioxan]-11-yl]phenyl-1,1,2,2,3,3,4,4,4-nonafluorbutan-1-sulfonat

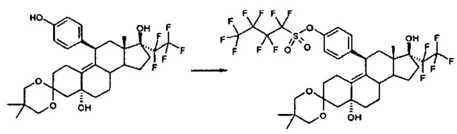

Zu einer Lösung von 9,16 g der unter 9b) beschriebenen Verbindung in 100 ml absolutem THF wurden bei 0°C 14,64 ml einer 1,6 molaren Lösung von n-Butyllithium in Hexan addiert. Man ließ 30 Minuten bei 0°C nachrühren und addierte dann langsam 5,62 ml Perfuorbutan-1-sulfonsäurefluorid. Anschließend ließ man weitere 1,5 Stunden bei 0°C nachrühren. Danach wurde das Reaktionsgemisch auf eine Mischung von 300 ml gesättigter Natriumhydrogencarbonatlösung und 90 ml 2 N Natronlauge gegossen. Es wurde 45 Minuten gerührt und dann mehrfach mit Ethylacetat extrahiert. Die vereinigten organischen Phasen wurden mit gesättigter Natriumchlorfdlösung gewaschen und über Natriumsulfat getrocknet. Das erhaltene Rohprodukt wurde durch Chromatographie an Kieselgel aufgereinigt. Man erhielt 10,1 g der Titelverbindung.
$^1H$-NMR (400 MHz, $CDCl_3$): δ= 7,28 d (2H); 7,18 d (2H); 4,42 s (1H); 4,34 dbr (1H); 3,50-3,58 m (3H); 3,42 d (1H); 1,05 s (3H); 0,86 s (3H); 0,50 s (3H).

d) (5R,8S,11R,13S,14S,17S)-5'.5',13-Trimethyl-11-[4'-(methylsulfanyl)biphenyl-4-yl]-17-(pentafluorethyl)-1,2,6,7,8,11,12,13,14,15,16,17-dodecahydrospiro[cyclopenta[a]phenanthren-3,2'-[1,3]dioxan]-5,17(4H)-diol

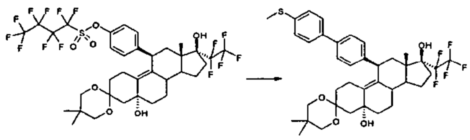

Zu einer Lösung von 1,2 g der unter 9c) beschriebenen Verbindung in einem Gemisch aus 12 ml Toluol und 6 ml Ethanol wurden 2 ml einer 2 molaren wässrigen Natriumcarbonatlösung, 131 mg Lithiumchlorid, 240 mg 4-(Methylthio)phenylboronsäure und 192 mg Tetrakis(triphenylphosphin)palladium addiert. Anschließend wurde 2 Stunden unter Rückfluss gekocht. Danach wurde das Reaktionsgemisch mit einem Gemisch aus Ethylacetat und Wasser versetzt. Man extrahierte mehrfach mit Ethylacetat. Die vereinigten organischen Phasen wurden mit gesättigter wässriger Natriumchloridlösung gewaschen, über Natriumsulfat getrocknet und im Vakuum eingeengt. Das Rohprodukt wurde durch Chromatographie an Kieselgel gereinigt. Man erhielt 927 mg der Titelverbindung.
$^1$H-NMR (300 MHz, $CDCl_3$): δ= 7,45-7,55 m (4H); 7,30 d (2H); 7,27 d (2H); 4,45 s (1H); 4,35 dbr (1H): 3,40-3,60 m (4H); 2,50 s (3H); 1,07 s (3H); 0,97 s (3H); 0,58 s (3H).

e) (11β,17β)-17-hydroxy-11-[4'-(methylsulfanyl)biphenyl-4-yl]-17-(pentafluorethyl)estra-4,9-dien-3-on

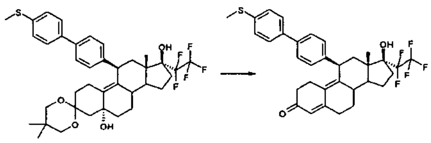

Analog zu Beispiel 1c) wurden aus 120 mg der unter 9d) hergestellten Verbindung durch Umsetzung mit halbkonzentrierte Schwefelsäure in Methanol 82 mg der Titelverbindung hergestellt.
$^1$H-NMR (400 MHz, $CDCl_3$): δ= 7,45-7,58 m (4H); 7,30 d (2H); 7,24 d (2H); 5,80 sbr (1H); 4,50 dbr (1H); 2,50 s (3H); 0,62 s (3H).

**Beispiel 10**

(11β,17δ)-17-hydroxy-11-[4'-(methylsulfonyl)biphenyl-4-yl]-17-(pentafluorethyl)estra-4,9-dien-3-on

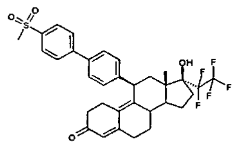

a) (5R,8S,11R,13S,14S,17S)-5',5',13-Trimethyl-11-[4'-(methylsulfonyl)biphenyl-4-yl]-17-(pentafluorethyl)-1,2,6,7,8,11,12,13,14,15,16,17-dodecahydrospiro[cyclopenta[a]phenanthren-3,2'-[1,3]dioxan]-5,17(4H)-diol

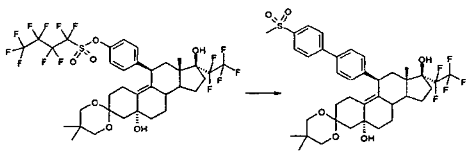

Analog zu Beispiel 9d) wurden aus 500 mg der unter Beispiel 9c) und (4-Methylsulfonylphenyl)boronsäure in Gegenwart von Tetrakis(triphenylphosphin)palladium, Lithiumchlorid, 2 molarer wässriger Natriumcarbonatlösung in einem Gemisch aus Toluol und Ethanol 256 mg der Titelverbindung hergestellt.

$^1$H-NMR (300 MHz, $CDCl_3$): δ= 8,03 d (2H); 7,80 d (2H); 7,58 d (2H); 7,39 d (2H); 4,48 s (1H); 4,45 dbr (1H); 3,45-3,65 m (4H); 3,12 s (3H); 1,10 s (3H); 0,91 s (3H); 0,62 s (3H).

b) (11β,17β)-17-hydroxy-11-[4'-(methylsulfonyl)biphenyl-4-yl]-17-(pentafluorethyl)estra-4,9-dien-3-on

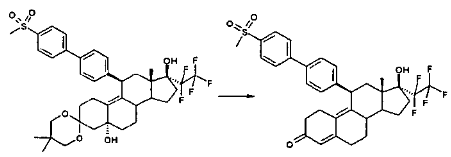

Analog zu Beispiel 1c) wurden aus 110 mg der unter 10a) hergestellten Verbindung durch Umsetzung mit halbkonzentrierte Schwefelsäure in Methanol 62 mg der Titelverbindung hergestellt.
$^1$H-NMR (400 MHz, $CDCl_3$): δ= 8,00 d (2H); 7,75 d (2H); 7,55 d (2H); 7,30 d (2H); 5,80 sbr (1H); 4,50 dbr (1H); 3,09 s (3H); 0,65 s (3H).

**Beispiel 11**

N-[{4'-[(11β,17β)-17-hydroxy-3-oxo-17-(pentafluorethyl)estra-4,9-dien-11-yl]biphenyl-4-yl}(RS)(methyl)oxido-$\lambda^6$-sulfanyliden]-4-methylbenzolsulfonamid

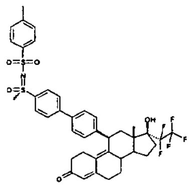

a) N-[{4'-[(5R,8S,11R,13S,14S,17S)-5,17-Dihydroxy-5',5',13-trimethyl-17-(pentafluorothyl)-1,2,4,5,6,7,8,11,12,13,14,15,16,17-tetradecahydrospiro[cyclopenta[a]phenanthren-3,2'-[1,3]dioxan]-11-yl]biphenyl-4-yl}(RS)(methyl)-$\lambda^4$-sulfanyliden]-4-methylbenzolsulfonamid

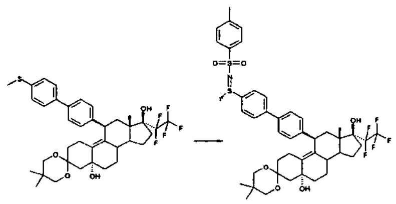

Analog zu Beispiel 7a) wurden aus 800 mg der unter Beispiel 9d) hergestellten Verbindung mit Chloramin-T-Tnhydrat® in Acetonitril 715 mg der Titelverbindung hergestellt.
$^1$H-NMR (300 MHz, $CDCl_3$): δ= 7,65-7,80 (6H); 7,47 d (2H); 7,30 d (2H); 7,18 d (2H); 4,45 s (1H); 4,39 dbr (1H); 3,40-3,60 m (4H); 2,87 (3H); 2,35 s (3H), 1,03 s (3H); 0,87 s (3H); 0,56 s (3H) (Mischung von Diastereomeren).

b) N-[(4'-[(5R,8S,11R,13S,14S,17S)-5,17-Dihydroxy-5',5',13-trimethyl-17-(pentafluorethyl)-1,2,4,5,6,7,8,11,12,13,14,15,16,17-tetradecahydrospiro[cyclopenta[a]phenanthren-3,2'-[1,3]dioxan]-11-yljbiphenyl-4-yl}(RS)(methyl)oxido-$\lambda^6$-sulfanyliden]-4-methylbenzolsulfonamid

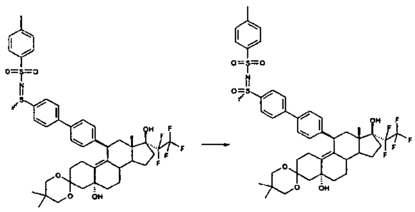

Analog zu Beispiel 7b) wurden aus 709 mg der unter Beispiel 11a) erhaltenen Verbindung durch Umsetzung mit 30%iger Wasserstoffperoxidlösung und Natriumcarbonat in einem Gemisch aus Acetonitril und Methanol 638 mg der Titelverbindung erhalten.
$^1$H-NMR (300 MHz, $CDCl_3$): δ= 8,04 d (2H); 7,87 d (2H); 7,78 d (2H); 7,50 d (2H); 7,35 d (2H); 7,27 d (2H); 4,46 s (1H); 4,40 dbr (1H); 3,40-3,60 m (4H); 3,46 s (3H); 2,39 s (3H); 1,07 s (3H); 0,87 s (3H); 0,56 s (3H) (Mischung von Diastereomeren).

c) N-[{4'-[(11β,17β)-17-hydroxy-3-oxo-17-(pentafluorethyl)estra-4,9-dien-11-yl]biphenyl-4-yl}(RS)(methyl)-oxido-λ$^6$-sulfanyliden]-4-methylbenzolsulfonamid

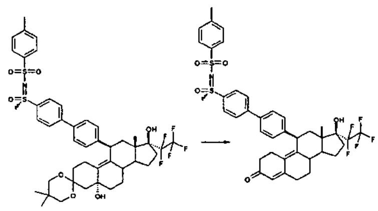

Analog zu Beispiel 1c) wurden aus 633 mg der unter 11b) hergestellten Verbindung durch Umsetzung mit halbkonzenbierte Schwefelsäure in Methanol 523 mg der Titelverbindung hergestellt.
$^1$H-NMR (300 MHz, $CDCl_3$): δ= 8,06 d (2H); 7,87 d (2H); 7,78 d (2H); 7,52 d (2H); 7,20-7,35 m (4H); 5,80 sbr (1H); 4,51 dbr (1H); 3,45 s (3H); 2,39 s (3H); 0,62 s (3H) (Mischung von Diastereomeren).

**Beispiel 12**

(11β,17β)-17-hydroxy-11-[4'-(RS-methylsulfonimidoyl)biphenyl-4-yl]-17-(pentafluorethyl)estra-4,9-dien-3-on

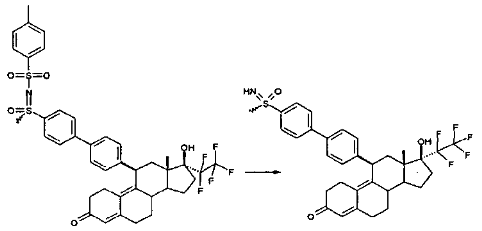

Analog zu Beispiel 8 wurden aus 500 mg der unter Beispiel 11c) hergestellten Verbindung durch Umsetzung mit konzentrierte Schwefelsäure in Chloroform 325 mg der Titelverbindung erhalten.
$^1$H-NMR (300 MHz, $CDCl_3$): δ= 8,07 d (2H); 7,74 d (2H); 7,55 d (2H); 7,30 d (2H); 5,80 sbr (1H); 4,51 dbr (1H); 3,15 s (3H); 0,64 s (3H) (Mischung von Diastereomeren).

**Beispiel 13**

(11β,17β)-17-hydroxy-17-(pentafluoroethyl)-11-(4'-sulfanylbiphenyl-4-yl)estra-4,9-dien-3-on

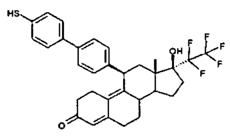

a) (5R,8S,11R,13S,14S,17S)-5',5',13-Trimethyl-17-(pentafluorethyl)-11-(4'-sultanylbiphenyl-4-yl)-1,2,6,7,8,11,12,13,14,15,16,17-dodecahydrospiro[cyclopenta[a]phenanthren-3,2'-[1,3]dioxan]-5,17(4H)-diol

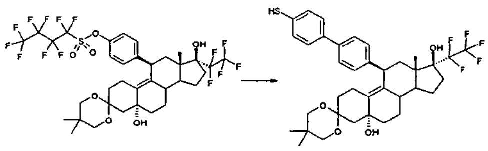

Analog zu Beispiel 9d) wurden aus 1 g der unter Beispiel 9c) beschriebenen Verbindung und (4-Mercaptophenyl)boronsäure in Gegenwart von Tetrakis(triphenylphosphin)palladium, Lithiumchlorid, 2 molarer wässriger Natriumcarbonatlösung in einem Gemisch aus Toluol und Ethanol 478 mg der Titelverbindung hergestellt.
$^1$H-NMR (400 MHz, $CDCl_3$): δ= 7,14-7,32 m (8H); 4,42 s (1H); 4,30 dbr (1H); 3,40-3,60 m (4H); 1,05 s (3H); 0,88 s (3H); 0,54 s (3H).

b) (11β,17β)-17-hydroxy-17-(pentafluoroethyl)-11-(4'-sulfanylbiphenyl-4-yl)estra-4,9-dien-3-on

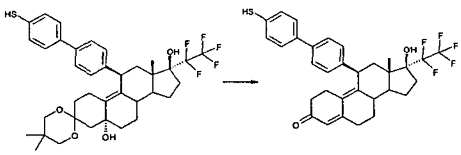

Analog zu Beispiel 1c) wurden aus 200 mg der unter 13a) hergestellten Verbindung durch Umsetzung mit halbkonzentrierte Schwefelsäure in Methanol 103 mg der Titelverbindung hergestellt.
$^1$H-NMR (400 MHz, $CDCl_3$): δ= 7,20-7,38 m (6H); 7,11d (2H); 5,78 sbr (1H); 4,42 dbr(1H): 0,61 s (3H).

Beispiel 14

4'-[(11β,17β)-17-hydroxy-3-oxo-17-(pentafluorethyl)estra-4,9-dien-11-yl]-N,N-dimethylbiphenyl-4-sulfonamid

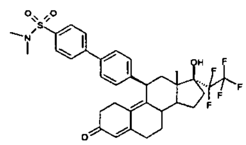

a) 4'-[(5R,8S,11 R,13S,14S,17S)-5,17-Dihydroxy-5',5',13-trimethyl-17-(pentafluorethyl)-1,2,4,5,6,7,8,11,12,13,14,15,16,17-tetradecahydrospiro[cyclopenta[a]phenanthren-3,2'-[1,3]dioxan]-11-yl]-N,N-dimethylbiphenyl-4-sulfonamid

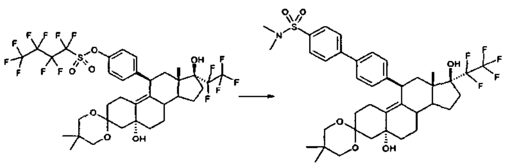

Analog zu Beispiel 9d) wurden aus 300 mg der unter Beispiel 9c) beschriebenen Verbindung und [4-[(Dimethylamino)sulfonyl]phenyl]boronsäure in Gegenwart von Tetrakis(triphenylphosphin)palladium, Lithiumchlorid, 2 molarer wässriger Natriumcarbonatlösung in einem Gemisch aus Toluol und Ethanol 235 mg der Titelverbindung hergestellt.
$^1$H-NMR (300 MHz, $CDCl_3$): δ= 7,83 d (2H); 7,73 d (2H); 7,52 d (2H); 7,33 d (2H); 4,47 s (1H); 4,39 dbr (1H); 3,40-3,60 m (4H); 2,75 s (6H); 1,06 s (3H); 0,88 s (3H); 0,57 s (3H).

b) 4'-[(11β,17β)-17-hydroxy-3-oxo-17-(pentafluorethyl)estra-4,9-dien-11-yl]-N,N-dimethylbiphenyl-4-sulfonamid

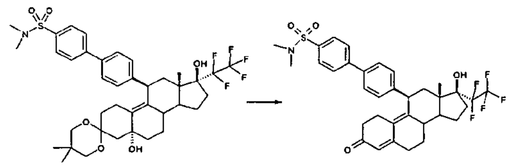

Analog zu Beispiel 1c) wurden aus 230 mg der unter 14a) hergestellten Verbindung durch Umsetzung mit halbkonzentrierte Schwefelsäure in Methanol 113 mg der Titelverbindung hergestellt.
$^1$H-NMR (400 MHz, $CDCl_3$): δ= 7,83 d (2H); 7,72 d (2H); 7,55 d (2H); 7,30 d (2H); 5,80 sbr (1H); 4,52 dbr (1H); 2,75 s (6H); 0,64 s (3H).

**Beispiel 15**

4-[(11β,17β)-17-Hydroxy-3-oxo-17-(pentafluorethyl)estra-4,9-dien-11-yl]-N,N-dimethylbenzolsulfonamid

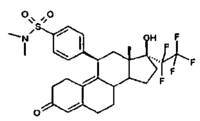

a)
4-[(5R,8S,11R,13S,14S,17S)-5,17-Dihydroxy-5',5',13-trimethyl-17-(pentafluorethyl)-1,2,4,5,6,7,8,11,12,13,14,15,16,17-tetradecahydrospiro[cyclopenta[a]phenanthren-3,2'-[1,3]dioxan]-11-yl]-N,N-dimethylbenzolsulfonamid

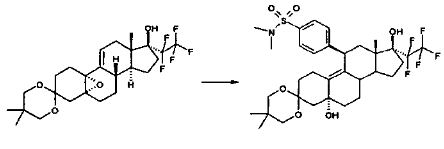

5,1 ml einer 2 molaren Lösung von Diisopropylmagnesiumchlorid in Diethylether wurden unter Kühlung (-10°C) mit 10 ml THF verdünnt. Danach wurden 8,12 ml einer 2,5 molaren Lösung von n-Butyllithium in Hexan bei -10°C über 30 Minuten hinzugetropft. Man ließ 2 Stunden nachrühren und addierte dann 15,1 mg CuCl. Nachdem weitere 5 Minuten nachgerührt worden war, wurde eine Lösung von 500 mg der unter Beispiel 1a) beschriebenen Substanz in 5 ml THF addiert. Man ließ 3 Stunden bei -10°C nachrühren und dann langsam auf 23°C erwärmen. Es wurde weitere 12 Stunden bei 23°C gerührt. Anschließend wurde gesättigte wässrige $NH_4Cl$-Lösung zum Reaktionsgemisch unter externer Kühlung

addiert. Man rührte weitere 30 Minuten nach und extrahierte dann mehrfach mit Ethylacetat. Die vereinigten organischen Phasen wurden mit gesättigter Natriumchloridlösung gewaschen und über Natriumsulfat getrocknet. Das Rohprodukt wurde durch Chromatographie an Kieselgel aufgereinigt. Man erhielt 214 mg der Titelverbindung.
$^1$H-NMR (300 MHz, $CDCl_3$): δ= 7,65 d (2H); 7,40 d (2H); 4,45 s (1H); 4,38 dbr (1H); 3,40-3,60 m (4H); 2,69 s (6H); 1,03 s (3H); 0,89 s (3H); 0,49 s (3H).

b) 4-[(11β,17β)-17-Hydroxy-3-oxo-17-(pentafluorethyl)estra-4,9-dien-11-yl]-N,N-dimethylbenzolsulfonamid

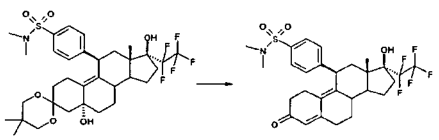

Analog zu Beispiel 1c) wurden aus 100 mg der unter 15a) hergestellten Verbindung durch Umsetzung mit halbkonzentrierte Schwefelsäure in Methanol 74 mg der Titelverbindung hergestellt.
$^1$H-NMR (400 MHz, $CDCl_3$): δ= 7,69 d (2H); 7,38 d (2H); 5,80 sbr(1H); 4,04 dbr (1H); 2,68 s (6H); 0,52 s (3H)

**Beispiel 16 Progesteronerezeptor-antagonistische Wirkung in stabilen Transfektanten humaner Neuroblastoma-Zellen (SK-N-MC Zellen) mit dem humanem Progesteron A- oder Progesteron B-Rezeptor und einem MTV-LUC Reporter Konstrukt**

SK-N-MC Zellen (humane Neuroblastoma Zellen), die stabil mit Plasmiden transfiziert sind, welche den humanen Progesteronrezeptor-B (pRChPR-B-neo) oder den humanen Progesteronrezeptor-A (pRChPR-A-neo) und ein Reporterkonstrukt (pMMTV-LUC) exprimieren, wurden 24 Stunden entweder in Abwesenheit (Negativkontrolle) oder in Gegenwart von steigenden Mengen der jeweiligen Testverbindung (0.01 nmol/l, 0.1 nmolll, 1 nmolll, 10 nmol/l, 100 nmol/l und 1 μmol/l) inkubiert, um die agonistische Wirksamkeit zu bestimmen. Als Positivkontrolle der Reportergeninduktion wurden die Zellen mit dem synthetischen Gestagen Promegeston (0.01 nmol/l, 0.1 nmol/l, 1 nmol/l, 10 nmol/l, 100 nmolll and 1 μmol/l) behandelt. Zur Bestimmung der antagonistischen Aktivität wurden die Zellen mit 0.1 nmol/l Promegeston und zusätzlich mit steigenden Mengen der jeweiligen Testverbindung (0.01 nmol/l, 0.1 nmol/l, 1 nmol/l, 10 nmol/l,100 nmol/l and 1 μmol/l) behandelt. Die Aktivität des Reportergens LUC (LUC = Luciferase) wurde in den Zelllysaten bestimmt und als RLU (relative light units) gemessen. Alle Messwerte werden angegeben als % Wirksamkeit und als $EC_{50}$ bzw. $IC_{50}$ Konzentrationen.
Neben der Testverbindung wurden als Vergleichsverbindungen 11β-(4-Acetylphenyl)-20,20,21,21,21-pentafluor-17-hydroxy-19-nor-17α-pregna-4,9-dien-3-on und 20,20,21,21,21-Pentafluor-17-hydroxy-11β-[4-(hydroxyacetyl)phenyl]-19-nor-17α-pregna-4,9-dien-3-on, sehr potente und daher bevorzugte Beispiele aus WO98/34947 und WO2008/058767, getestet.

a) agonistische Aktivität:

Keine der genannten Testverbindungen zeigt eine agonistische Aktivität.

b) antagonistische Aktivitat:

Alle genannten Verbindungen zeigen eine 100%ige antagonistische Wirksamkeit.
Die antagonistische Wirkstärke der Verbindungen ist in Tabelle 1 zusammengefasst.

| Verbindung | Progesteron Rezeptor-A (PR-A) | | Progesteron Rezeptor-B (PR-B) | |
|---|---|---|---|---|
| | **Potenz** $IC_{50}$ [nmol/l) | **Wirksamkeit** [%] | **Potenz** $IC_{50}$ [nmol/l] | **Wirksamkeit** [%] |
| 11β-(4-Acetylphenyl)-20,20,21,21,21-pentafluor-17-hydroxy-19-nor-17α-pregna-4,9-dien-3-on | 0.014 | 100 | 0.02 | 100 |

(fortgesetzt)

| Verbindung | Progesteron Rezeptor-A (PR-A) | | Progesteron Rezeptor-B (PR-B) | |
|---|---|---|---|---|
| | **Potenz** $IC_{50}$ [nmol/l) | **Wirksamkeit** [%] | **Potenz** $IC_{50}$ [nmol/l] | **Wirksamkeit** [%] |
| 20,20,21,21,21-Pentafluor-17-hydroxy-11β-[4-(hydroxyacatyl)phenyl]-19-nor-17α-pregna-4,9-dien-3-on dien-3-on | 0.18 | 100 | 0.28 | 100 |
| Beispiel 1 | 0.011 | 100 | 0.012 | 100 |
| Beispiel 2 | 0.01 | 100 | 0.01 | 100 |
| Beispiel 3 | 0.11 | 100 | 0.12 | 100 |
| Beispiel 4 | 0.096 | 100 | 0.087 | 100 |
| Beispiel 5 | 0.1 | 100 | 0.09 | 100 |
| Beispiel 6 | 0.2 | 100 | 0.23 | 100 |
| Beispiel 7 | 1.0 | 100 | 0.8 | 100 |
| Beispiel 8 | 0.9 | 100 | 0.9 | 100 |
| Beispiel 9 | 0.01 | 100 | 0.01 | 100 |
| Beispiel 10 | 0.011 | 100 | 0.013 | 100 |
| Beispiel 11 | 0.01 | 100 | 0.01 | 100 |
| Beispiel 12 | 0.08 | 100 | 0.08 | 100 |
| Beispiel 13 | 0.072 | 100 | 0.072 | 100 |
| Beispiel 14 | 0.01 | 100 | 0.01 | 100 |
| Beispiel 15 | 0.1 | 100 | 0.2 | 100 |

Beispiel 17 Abortivtest an weiblichen Ratten

Die Wirkung von Progesteron und des Progesteron Rezeptors sind für eine erfolgreiche Schwangerschaft bzw. Trächtigkeit bei Säugetieren grundlegende Voraussetzung. Die Progesteronantagonistische Wirkung der erfindungsgemäßen Verbindung und der Verbindungen der Vergleichsbeispiele 1 und 8 wurde an trächtigen Ratten (6 Ratten pro Gruppe) an Tag 5 bis 7 post coitum unter herkömmlichen Haltungs- und Fütterungsbedingungen getestet. Nach erfolgreicher Anpaarung, wurden die trächtigen Tiere (Vorhandensein von Spermien im Vaginalabstrich an Tag 1 der Schwangerschaft = d1 p.c.) randomisiert und auf die Behandlungs- und Kontrollgruppe aufgeteilt. Die Tiere erhielten dann subkutan oder oral je 0,15; 0,5; 1,5 oder 5 mg/kg der Testverbindung oder 1,0 ml/kg Vehikel (Benzylbenzoat/Rhizinusöl: 1+4 [vlv]) täglich von Tag 5 bis Tag 7 (d5 -d7 p.c.).

Die Autopsie wurde an Tag 9 (d9 p.c.) durchgeführt. Als Kenngröße der Progesteronrezeptorantagonistischen Wirkung wurde der Uterus auf das Vorhandensein von Nidationsstellen untersucht. Dabei wurde das völlige Fehlen, aber auch das Vorhandensein pathologischer, hämorrhagischer oder sonst abnormer Nidationsstellen an Tag 9 (d9 p.c.) als Abort gewertet. Die Ergebnisse der Tests sind in Tabelle 3 dargestellt.

Tab. 3: Ergebnisse an der Ratte (Terminierung der frühen Schwangerschaft)

| Testverbindung nach | Tagesdosis [mg/kg] s.c. oder p.o. | Abortrate [%] |
|---|---|---|
| Vehikel | | 0 |
| Beispiel 1 (11β,17β)-17-hydroxy-11-[4-(methylsulfanyl)phenyl]-17-(pentafluorethyl)estra-4,9-dien-3-on | 0,5 | 80 |
| | 1,5 | 100 |
| | 5,0 | 100 |

(fortgesetzt)

| Testverbindung nach | Tagesdosis [mg/kg] s.c. oder p.o. | Abortrate [%] |
|---|---|---|
| Beispiel 4 (11β,17β)-17-hydroxy-11-[4-(methylsulfonyl)phenyl]-17-(pentafluorethyl)estra-4,9-dien-3-on | 0,15 | 40 |
| | 0,5 | 100 |
| | 1,5 | 100 |
| | 5,0 | 100 |
| Beispiel 8 (11β,17β)-17-hydroxy-11-[4-(RS-methylsulfonimidoyl)-phenyl]-17-(pentafluor-ethyl)estra-4,9-dien-3-on | 0,15 | 40 |
| | 0,5 | 100 |
| | 1,5 | 100 |
| | 5,0 | 100 |

**Beispiel 18) Metabolische Stabilität von (11β,17β)-17-hydroxy-11-[4-(methylsulfonyl)phenyl]-17-(pentafluorethyl)estra-4,9-dien-3-on und (11β,17β)-17-hydroxy-11-[4'-(methylsulfonyl)-biphenyl-4-yl]-17-(pentafluorethyl) estra-4,9-dien-3-on in humanen Lebermikrosomen (HLM)**

Es wurden isolierte humane Lebermikrosomen (HLM) zur Beurteilung der metabolischen Stabilität von Verbindungen der Beispiele eingesetzt.
Die Inkubationen wurden mit 2.4 ml HLM-lösung (0.5 mg/ml Proteingehalt), 30 μl der Testverbindung (finale Konzentration 1 μM) und 0.6 ml des Cofaktorgemisches (=NADPH-generierendem System aus 3 IU Glucose-6-phosphatdehydrogenase, 14.6 mg Glucose-6-phosphat, 1.2 mg NADP) bei 37°C in 100 mM Phosphatpuffer bei pH 7.4 durchgeführt. Es werden zu 6 Zeitpunkten (2 - 60 min) Proben entnommen, mit gleichem Volumen Methanol gefällt und der Widerfund der eingesetzten Testsubstanzen im Überstand mittels LC-MS/MS-Analytik ermittelt. Aus der daraus ermittelten Halbwertzeit des Substanzabbaus kann man die intrinsische Clearance der Substanz im Lebermikrosomenansatz berechnen. Mit Hilfe dieser kann dann unter Zuhilfenahme verschiedener physiologischer Kenngrößen nach dem well-stirred Modell eine (metabolische) *in vivo* Clearance in Bezug auf Phase I Reaktionen vorhersagt werden. Die entsprechend für die Testverbindungen (11β,17β)-17-hydroxy-11-[4-(methylsulfonyl)phenyl]-17-(pentafluorethyl)estra-4,9-dien-3-on und (11β,17β)-17-hydroxy-11-[4'-(methylsulfonyl)biphenyl-4-yl]-17-(pentafluorethyl)-estra-4,9-dien-3-on vorhergesagte (metabolische) *in vivo* Clearance im Menschen war mit 0.1 L/h/kg bzw. < 0.01 L/h/kg sehr niedrig.

**Beispiel 19) Permeation von (11β,17β)-17-hydroxy-11-[4-(methylsulfonyl)phenyl]-17-(pentafluorethyl)estra-4,9-dien-3-on in Caco-2 Zellen.**

Für die Permeationsstudien wurde die Die Caco2 Zellen mit einer Zellzahl von 300000 Zellen/ml auf Transwell Clear Filterineinsätzen (Polyester; Porengröße 0.4 μm) in 12 Loch- Zellkulturplatte für mindestens 14 Tage in Zellkulturmedium (1.5 ml) bei 37 °C, 5 % CO2 und 95 % Luftfeuchtigkeit kultiviert. Vor dem Versuchs wurde zur Überprüfung der "Dichtigkeit" des Zellmonolayers der Transepitheliale Widerstand (TEER-Wert) bestimmt, welcher größer als 300 Ω $cm^2$ sein muß. Anschließend wurden das Zellkulturmedium gegen warmen Transportpuffer (0,5 ml apikal, 1,5 ml basolateral) ausgetauscht und die Zellen 5 min darin equilibriert. Der Permeabilitätsversuch wurde in Doppelbestimmung bei einer Substanzkonzentration von 2 μM durchgeführt. Zu Beginn des Experimentes wurde 100 μl (Ap0min) aus dem apikalen Kompartiment entnommen und sofort mit 100 μl eiskalter Stopplösung versetzt. Die Filter wurden anschließend bei 37 °C unter sanftem Schütteln für 90 min inkubiert, anschließend wieder 100 μl aus der apikalen Seite (Ap90min) und 400 μl aus der basolateralen Seite (Bas90min) entnommen und mit jeweils gleichen Volumen Stopplösung versetzt. Nach weiterer Verdünnung der Proben mit dem 4fachen Volumen Stopplsg/Transportpuffer (1+1) wurden sie über Nacht bei -20°C ausgefällt und der Überstand mittels LCMS/MS analytisch vermessen. Mittels u.g. Formel wurde der Papp Wert der Substanzen berechnet.

$$Papp = \frac{V_{res}}{A \cdot C_{t0,don}} \bullet \frac{\Delta C_{res}}{\Delta t}$$

$V_{res}$: Puffervolumen auf der Rezeptorseite; A: Filterfläche = 1 $cm^2$; $C_{t0,\ don}$: Substanzkonzentration auf der Donorseite; $AC_{res}/\Delta t$: Änderung der Substanzkonzentration über die Zeit auf der Rezeptorseite
(11 β,17β)-17-hydroxy-11-[4-(methylsulfonyl)phenyl]-17-(pentafluorethyl)estra-4,9-dien-3-on zeigte in diesem Assay eine sehr hohe Permeation von 104 nm/s.

**Beispiel 20) Untersuchung der Wirkung auf das Herz-Kreislauf-System (inkl. EKG) narkotisierter Beagle Hunde**

(11β,17β)-17-Hydroxy-11-[4-(methylsulfonyl)phenyl]-17-(pentafluorethyl)estra-4,9-dien-3-on wurde gelöst in einem Gemisch aus PEG 400 und HP-β-CD (60% PEG 400, 40% HP-β-CD 30%ig) intravenös narkotisierten weiblichen Beagle Hunden verabreicht. Das Körpergewicht der Hunde betrug > 9 kg. Pro Gruppe wurden 3 Hunde behandelt und zusätzlich 3 Hunde in der Kontrollgruppe. Es wurde in 3 konsekutiven Infusionen über jeweils 30 Minuten 0,1; 0,33 und 1 mg/kg der Substanz gegeben. Die maximale Menge an Vehikel betrug 0,4 ml pro kg über 30 Minuten. Den Tieren wurden zu verschiedenen Zeitpunkten Blutproben entnommen. Die höchsten Plasmalevel (Durchschnitt über alle 3 Tiere) betrug am Ende der dritten Infusion 1650 ng/ml.

Im getesteten Dosisbereich wurden im Vergleich zur Kontrolle keine biologisch relevanten Wirkungen auf das Herz-Kreislauf-System (Pulmonal arterieller Blutdruck, Systemisch arterieller Blutdruck, Herzfrequenz, EKG) beobachtet.

## Patentansprüche

1. (11β, 17β)-17-Hydroxy-11-[4-(methylsulphonyl)phenyl]-17-(pentafluoroethyl) estra-4,9-dien-3-on der Formel

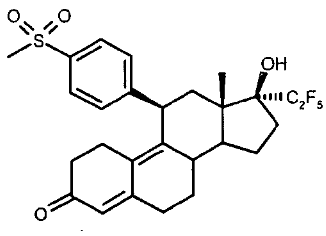

oder Salze davon.

2. Arzeimittel enthaltend eine verbindung, wie im Anspruch 1 definiert, in Kombination mit einem inerten, nicht toxischen, pharmazeutisch geeigneten Hilfsstoff.

## Claims

1. (11β,17β)-17-Hydroxy-11-[4-(methylsulphonyl)phenyl]-17-(pentafluoroethyl)estra-4,9-dien-3-one of formula

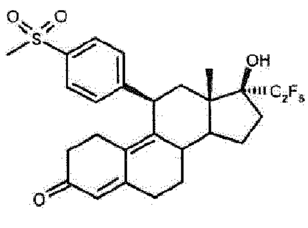

or salts thereof.

2. Medicinal product containing a compound as defined in Claim 1, in combination with an inert, non-toxic, pharmaceutically suitable excipient.

## Revendications

1. (11β,17β)-17-hydroxy-11-[4-(méthylsulfonyl)-phényl]-17-(pentafluoroéthyl)estra-4,9-dién-3-one de formule

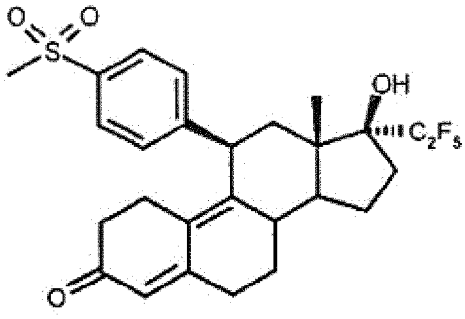

ou ses sels.

2. Médicament contenant un composé tel que défini dans la revendication 1, en association avec un adjuvant inerte, non toxique, pharmaceutiquement approprié.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 57115 A
- WO 9834947 A
- WO 2008058767 A
- WO 9615794 A, Spicer
- WO 9603130 A, Stöckemann
- EP 2009003249 W, Möller
- WO 98007740 A
- WO 9933855 A
- WO 03045972 A
- WO 0147490 A
- WO 06010097 A


### In der Beschreibung aufgeführte Nicht-Patentliteratur


- **FUHRMANN et al.** *J. Med. Chem.,* 2000, vol. 43, 5010-5016
- *Tetrahedron Lett,* 1985, vol. 26, 2069-2072
- **HOFFMANN J et al.** *J Natl Cancer Inst,* 2004, vol. 96, 210-218
- *Tetrahedron Lett.,* 1985, vol. 26, 2069-2072